# EUROPEAN PATENT SPECIFICATION

(11) **EP 2 170 414 B1**
(45) Date of publication and mention of the grant of the patent: **29.08.2012**
(21) Application number: 08763426.7
(22) Date of filing: 01.07.2008
(51) Int. Cl.: A61L 24/10, A61L 31/04, A61L 24/04

(54) **KITS, FORMULATIONS AND SOLUTIONS HAVING ENZYMATICALLY-PERMISSIVE AMOUNTS OF VISUALIZATION AGENTS AND USES THEREOF**
KITS, FORMULIERUNGEN UND LÖSUNGEN MIT ENZYMATISCH PERMISSIVEN MENGEN VON VISUALISIERUNGSMITTELN UND IHRE VERWENDUNGEN
TROUSSES, FORMULATIONS ET SOLUTIONS CONTENANT DES QUANTITÉS D'AGENTS DE VISUALISATION PERMISSIVES PAR VOIE ENZYMATIQUE ET LEURS UTILISATIONS

(30) Priority: 02.07.2007 EP 07111565; 02.07.2007 US 929534 P; 09.04.2008 US 71031
(43) Date of publication of application: 07.04.2010
(60) Divisional application: 12172045.2
(73) Proprietor: Omrix Biopharmaceuticals Ltd., 76106 Rehovot (IL)
(72) Inventor: NUR, Israel, Moshav Timmorim (IL); MEIDLER, Roberto, 58444 Holon (IL); BAR, Liliana, 76247 Rehovot (IL)
(74) Representative: James, Anthony Christopher W.P.
(86) International application number: PCT/IB2008/052636
(87) International publication number: WO 2009/004573

(56) References cited:
- WO-A-91/04073
- WO-A-97/15188
- WO-A-03/047530
- US-A1- 2003 077 272
- US-A1- 2005 049 178
- DATABASE WPI Week 200303 Thomson Scientific, London, GB; AN 2003-032422 XP002458007 & JP 2002 104996 A (MOCHIDA PHARM CO LTD) 10 April 2002 (2002-04-10) cited in the application

## Description

### Field of the invention

The invention relates to a fibrin-glue kit for preparing a fibrin-glue formulation comprising an enzymatically-permissive concentration of a visualization agent.

### Background of the invention

Fibrin glue is typically a blood product obtained from either commercial sources or some regional blood transfusion centers. Components that are commonly used in the preparation of fibrin glues are fibrinogen, thrombin, Factor VIII, Factor XIII, fibronectin, vitronectin and von Willebrand factor (vWF).

Fibrin glue is formed by an enzymatic reaction involving inter alia, fibrinogen, thrombin and Factor XIII. The thrombin converts the fibrinogen to fibrin by enzymatic action at a rate determined by the concentration of thrombin. Factor XIII, is typically present in the fibrinogen component of the glue and is an enzyme of the blood coagulation system that cross-links and stabilizes the fibrin clot. This process bypasses most of the steps of normal coagulation and mimics its last phase. Some manufacturers add anti-proteolytic agents to the fibrin glue formulation (as described in WO-A-93/05822) or specifically remove the plasminogen in order to stop or delay the fibrinolysis (as described in US-B-5,792,835 and US-B-7,125,569).

Numerous uses of fibrin glue in various medical fields have been reported, including use as a sealant, hemostatic agent, anti-adhesive and in a variety of laparoscopic surgeries. However, fibrin glues result in a transparent film which may be notable in open surgery when the lighting is optimal, but could be rather unnoted in certain laparoscopic surgeries. Therefore, it would be advantageous to use dyed fibrin glue which will enable the user to asses the thickness of the applied material and improve its visibility during surgery.

The US-B-7,009,034 discloses a composition suitable for coating a tissue of a patient comprising polymers, small molecule "crosslinkers" which remain in the cross-linked polymer and a visualization agent. The disclosed polymers can be synthetic or natural. The natural polymers mentioned in the US-B-7,009,034 are collagen, fibrinogen, albumin, and fibrin, polysaccharides, or glycosaminoglycans. The description of US-B-7,009,034 is silent on Factor XIII and/or thrombin. The Examples show that addition of a visualization agent to the composition of US-B-7,009,034 at a high concentration of 1.25% did not cause any unacceptable changes in gelation times. According to the description higher concentration than 1.25% may be used, up to the limit of the solubility of the visualization agent in the final mixture.

The following patent applications do not disclose or suggest any undesired effects of dye addition on thrombin activity.

US-A-2003/0077272 discloses proteinaceous gels having visualization agents. Disclosed are gels comprising fibrinogen, thrombin and small molecule "cross-linkers" which remain in the cross-linked polymer. Few examples mention possible preparations of fibrinogen and Factor XIII composition, using hydrogels and the formation of fibrin adhesive by thrombin. These examples disclose addition of a fluorescent dye to the fibrinogen solution at a concentration of 0.0002-0.02% and are silent on the final concentration in the fibrin glue after the fibrinogen is mixed with the thrombin solution.

US-A-2005/0049178 discloses an agent for occlusion of blood vessels comprising a physiological safe dye. The dye enables staining of the embolized blood vessels. A preferred agent is a liquid fibrinogen solution which can be used in cooperation with a liquid thrombin preparation and Factor XIII. The patent application does not disclose any particular concentration of dye.

JP-A-2002104996 discloses a hemostatic composition which contains an active ingredient, such as thrombin, and a coloring matter which can avoid misuse in medical treatment, i.e., confusion between topical applications and injections. The dye is present in the composition in a wide range of 0.0001 to 1%.

WO-A-91/04073 discloses a photodynamic therapy which utilizes an energy absorbing material such as dye and a soldering agent such as fibrinogen or fibrin glue to achieve welding of a tissue. According to the invention the dye is considered as a chemically active ingredient and welding occurs only when a sufficient amount of energy is imparted to the energy absorbing material using an energy source such as a laser.

US-A-5,292,362 is directed to a composition including at least one natural or synthetic peptide and at least one support material which may be activated by energy to form a bond or coating. Fibrinogen and thrombin are mentioned among many peptides which can be employed as the first component of the composition. The second component contributes to the first component by producing an improved degree of inter-relationship among the molecules of the first component. According to the description the composition may also include endogenous or exogenous chromophores. The dyes are present in the composition in a broad range of from about 0.01% by weight to 50% by weight based on the total weight of the composition. WO 97/15188 discloses a reinforced biological sealant comprising a fibrinogen composition and a thrombin composition, which may optionally comprise a colored indicator substance.

WO 03/047530 discloses fibrin sealants which may comprise dyes in an amount of from 0.01% by weight to 50% by weight.

### Summary of the invention

Fibrin glues are used increasingly in surgery to reduce bleeding, and adhesions, to sealing or filling surfaces and/or improve wound healing. The up-to-date fibrin glue formulations are colorless; therefore applying the preparation to the oozing site remains difficult to control. Addition of a visualization agent improves the application targeting qualities of the fibrin glue, e.g. simplifies locating the application area, enables the user to asses the thickness of the applied material and improves the visibility of the applied material. However, it was found according to the present invention that addition of increased concentrations of visualization agents to the fibrin glue formulation affects the activity of thrombin.

Also, it was found according to the invention that different visualization agents affected differently thrombin clotting activity or clot formation.

The present invention solves this problem since the visualization agent is added at a concentration which is permissive to the activity of thrombin or of any other enzyme capable of forming fibrin when it reacts with fibrinogen.

Advantageously, according to the invention the visualization agent is added to the fibrin glue or a component thereof at a concentration that is low enough to be enzymatically-permissive but which is sufficient to clearly stain the application site in a manner that the area can be located, the thickness of the applied material can be assessed and/or the applied material can be distinguished.

The invention provides a fibrin glue kit for preparing a fibrin glue formulation for application to a surface of a body part of a patient, as defined in claim 1.

The proteolytic enzyme of the invention is thrombin.

The kit can further comprise a catalyst capable of inducing cross-linking of fibrin.

In one embodiment of the present invention, the catalyst is a transglutaminase such as Factor XIII.

In another further embodiment of the invention, Factor XIII is incorporated in the component comprising the fibrinogen.

The fibrin glue kits according to the invention can be used for promoting blood coagulation, for preventing and/or reducing of adhesions, for use in laparoscopic surgery and/or for sealing or filling surfaces.

### Brief description of the drawings

The features, aspects, and advantages of the present invention will become better understood with regard to the following description, examples, claims, and the following figures.

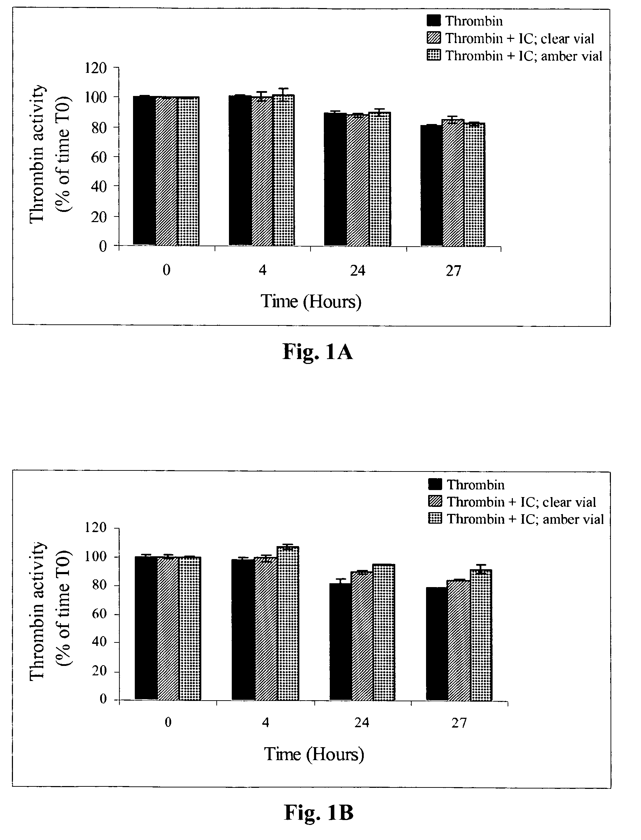

**Fig. 1A-1B****:** shows thrombin activity in two different batches (A and B) following prolonged incubation of the dyed and non-dyed thrombin solution with or without exposure to day light. The results obtained are expressed as a fold decrease in thrombin clotting activity as compared to the activity of the sample at T0 (100%). IC- indigo carmine.

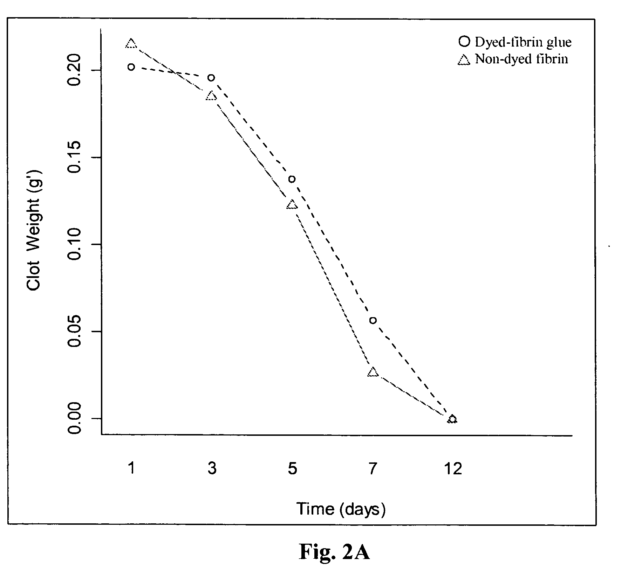

**Fig. 2A-2B****:** shows the clot weight (A) and the clottable protein amount (B) of both dyed and non-dyed fibrin glue at various time points. The experiment was carried out in an in vivo setting and the measurements were carried out 1, 3, 5, 7 and 12 days after the extraction of the clot remains from the rat abdomen. Each point represents the mean value of triplicate determinations.

### Detailed description of the invention

The invention relates to a fibrin-glue kit comprising an enzymatically-permissive concentration of visualization agent. The term "fibrin glue" as used herein includes a fibrin sealant, fibrin film, fibrin network, fibrin lattice, fibrin mesh, fibrin greed and fibrin gel.

The invention is based on findings of the invention demonstrating undesired effects of dye addition on thrombin activity. For example, it was found according to the present invention that addition of increased concentrations of visualization agents to a thrombin solution affects thrombin clotting activity or clot formation when applied to a fibrinogen solution. Also, it was found that different visualization agents affected differently thrombin clotting activity or clot formation. In addition, it was found that exposure of a visualization agent to light may increase the undesired effects of the agent on thrombin activity. Therefore, the present invention provides colored fibrin-glue or solution of a proteolytic enzyme capable of forming fibrin when it reacts with fibrinogen and methods for improving the application targeting qualities of the glue without substantially changing the clotting activity and/or the mechanical properties of the formed glue. Advantageously, according to the invention the visualization agent is added to the fibrin glue or a component thereof at a concentration that is low enough to be enzymatically-permissive but is sufficient to clearly stain the application site in a manner that the area can be located, the thickness of the applied material can be assessed and/or the applied material can be distinguished.

In one aspect, the invention relates to a fibrin glue kit as defined in claim 1.

In one embodiment of the invention each of the components of the glue are in separated recipients such as syringes which are emptied simultaneously and a fibrin clot is formed when the components are mixed.

The concentration of the fibrinogen in the kits of the invention can be in the range of from about 15 to about 150 mg/ml, of 40 to about 100 mg/ml, or from about 40 to about 60 mg/ml.

The visualization agents disclosed herein can be blood compatible, i.e. a color which provides contrast when applied on a bleeding surface such as blue.

The preferred spectrum of the visualization agent is the spectrum corresponding to a dye visible to the human eye. Visualization agents of the invention are methylene blue and indigo carmine. In one embodiment of the invention the visualization agent is methylene blue. In another embodiment of the invention the visualization agent is indigo carmine.

The word "enzyme" in the term "enzymatically-permissive" refers to the proteolytic enzyme which is capable of forming fibrin when it reacts with fibrinogen. By "enzymatically-permissive concentration of visualization agent" it is meant that the visualization agent is present in the proteolytic solution or in the fibrin glue at a concentration which allows solubility and which permits to retain from about 50 to about 100% of the proteolytic enzyme clotting activity in the absence of the visualization agent, i.e. the remaining proteolytic enzyme clotting activity following addition of the visualization agent is in the range of from about 50 to about 100% of the initial activity. In one embodiment of the invention, the remaining clotting activity after addition of the visualization agent is in the range of from about 90 to about 100%.

Thrombin clotting activity can be measured directly, for example, by the modified, European Pharmacopeia Assay (0903/1997) procedure and/or indirectly, such as measuring migration length on a slanted surface (or drop test model) as described in the Examples below, or by any other method known in the art.

In the present invention, the proteolytic enzyme is thrombin. The thrombin solution typically comprises thrombin and calcium chloride. The initial concentration of thrombin prior to the addition of the visualization agent can be in the range of from about 2 to about 4,000 IU/ml, or in the range of from about 800 to about 1200 IU/ml. Calcium chloride concentration in the solution can be in the range of from about 2 to about 6.2 mg/ml, or in the range of from about 5.6 to about 6.2 mg/ml, such as in the concentration of 5.88 mg/ml. The thrombin solution may comprise also excipients. As used herein the terms "excipient" refers to an inert substance which is added to the pharmaceutical composition. Examples of excipients include, but are not limited to, human albumin, mannitol, sodium acetate and water for injection. The human albumin in the solution can be in the range of from about 2 to about 8 mg/ml. Mannitol can be in the concentration range of from about 15 to about 25 mg/ml. Sodium acetate can be also added in the solution in the range of from about 2 to about 3 mg/ml.

In one embodiment of the invention, the kit of the invention further comprises a catalyst capable of inducing cross-linking of fibrin.

The term "catalyst" generally refers to a substance which presence increases the rate of a chemical reaction and remains substantially unchanged after completion of the respective chemical reaction in which it is involved. The catalyst can be an enzyme, e.g. transglutaminase. In one embodiment of the invention, the catalyst is Factor XIII. The catalyst capable of inducing cross-linking of fibrin can be included in the component comprising the fibrinogen, in the thrombin component and/or can be in a separated component. In one embodiment of the invention, Factor XIII is present in the component comprising the fibrinogen.

The fibrinogen, the catalyst, the proteolytic enzyme and/or the visualization agent is provided in the kit of the invention as a solution. The solution can be in frozen state. The kit can include instructions for use.

The solution can be prepared with a pharmaceutically acceptable carrier. The term "pharmaceutically acceptable carrier" refers to a carrier which is suitable for administration to a human or other animal. The term "carrier" denotes an ingredient with which the components are combined to facilitate the application of the composition in a manner such that the desired efficiency is substantially retained.

In one embodiment of the invention, one component is comprised of fibrinogen, and a co-stabilizer such as arginine, lysine or 4-(amino methyl)-cyclo-hexane-carboxylic acid (tranexamic acid) and combinations thereof.

According to the invention the fibrin glue components can be prepared from initial blood composition. The blood composition can be whole blood or blood fractions, i.e. a product of whole blood such as plasma. The fibrinogen component, the proteolytic enzyme and the catalyst can be autologous, human including pooled plasma, or of non-human source.

In one embodiment of the invention, the fibrinogen component is comprised from a biologically active component (BAC) which is a solution of proteins derived from blood plasma which can further comprise tranexamic acid and arginine or lysine or mixtures or arginine and lysine, or their pharmaceutically acceptable salts. BAC can be derived from cryoprecipitate, in particular concentrated cryoprecipitate. The term "cryoprecipitate" refers to a blood component which is obtained from frozen plasma prepared from whole blood. A cryoprecipitate can be obtained when frozen plasma is thawed in the cold, typically at a temperature of 0-4°C, resulting in the formation of precipitated supernatant that contains fibrinogen and factor XIII. The precipitate can be collected, for example by centrifugation. The solution of BAC comprises further Factor VIII, fibronectin, von Willebrand factor (vWF), vitronectin, etc. for example as described in US-B-6,121,232 and WO9833533. Preferably, the composition of BAC can comprise stabilizers such as tranexamic acid and arginine hydrochloride. Typically, the amount of fibrinogen in BAC is in the range of from about 40 to about 60 mg/ml. The amount of tranexamic acid in the solution of BAC can be from about 80 to about 110 mg/ml. The amount of arginine hydrochloride can be from about 15 to about 25 mg/ml.

Optionally, the solution is buffered to a physiological compatible pH value. The buffer can be composed of glycine, sodium citrate, sodium chloride, calcium chloride and water for injection as a vehicle. Glycine can be present in the composition in the amount of from about 6 to about 10 mg/ml, the sodium citrate can be in the range of from about 1 to about 5 mg/ml, sodium chloride can be in the range of from about 5 to about 9 mg/ml and calcium chloride can be in the concentration of about 0.1-0.2 mg/ml.

In another embodiment, the concentration of plasminogen and plasmin in the BAC composition is lowered to equal or less than 15 µg/ml like for example 5 µg/ml or less plasminogen using a method as described in US-B-7,125,569 and WO02095019

It is also possible that the fibrin glue kit comprises components which encourage the formation of the clot, such as Ca²⁺, Factor VIII, fibronectin, vitronectin, von Willebrand factor (vWF) which can be provided as a separate component or formulated with the fibrin glue components.

The protein components of the fibrin glue can be prepared by recombinant methods. It is also possible that part or all of the fibrin glue protein components are prepared by recombinant methods.

Fibrin glue components derived from blood compositions are typically purified from infective particles. The purification procedure can be carried out by nanofiltration, solvent/detergent treatment, heat treatment such as, but not limited to, pasteurization, gamma or UVC (< 280 nm) irradiation, or by any other method known in the art. The term "infective particle" refers to a microscopic particle, such as micro-organism or a prion, which can infect or propagate in cells of a biological organism. The infective particles can be viral particles.

Viral inactivation procedure can be carried out by adding a molecule to the composition or blood fraction prior to and/or during the purification procedure. The added molecules and their products can be removed by gravitation, column chromatography or any other method known in the art.

The removal of infective particles can be carried out by nanofiltration or by selective absorption methods such as affinity, ion exchange or hydrophobic chromatography. A multi-step viral inactivation procedure can be carried out. For example, the composition can be subjected to solvent/detergent treatment, heat treatment, selective chromatography and nanofiltration.

The term "surface of a body part of a patient" refers to an external surface of the skin that can be seen by unaided vision and to a surface of an internal body part which is a part of the internal anatomy of an organism. External surfaces include, but are not limited to, the skin of the face, throat, scalp, chest, back, ears, neck, hand, elbow, hip, knee, and other skin sites. Examples of internal body parts include, but are not limited to, body cavity or anatomical opening that are exposed to the external environment and internal organs such as the nostrils; the lips; the ears; the genital area, including the uterus, vagina and ovaries; the lungs; the anus; the spleen; the liver; and the cardiac muscle. The surface can be a bleeding or a non-bleeding site. The fibrinogen and the proteolytic enzyme are in solution and therefore need to be in separated components. The visualization agent in the kit of the invention can be formulated with one of the components before formation of the fibrin clot and/or can be in a separated component e.g. dissolved in an acceptable carrier which is suitable for application to the human or animal body.

The visualization agent is present together with the proteolytic enzyme.

In one embodiment of the invention, methylene blue dyed proteolytic enzyme solution is in a concentration range of from 0.01 to 0.05% and the final concentration in the cross-linked fibrin glue is in the range of from 0.005 to 0.025%.

In another embodiment of the invention, indigo carmine dyed proteolytic enzyme solution is in a concentration range of from 0.01 to 0.02%, for example at a concentration of about 0.015% and the final concentration in the cross-linked fibrin glue is in the range of from 0.005 to 0.01%, for example at a concentration of about 0.0075%. In another further embodiment of the invention, indigo carmine dyed proteolytic enzyme solution is in a concentration of 0.016% and the final concentration in the cross-linked fibrin glue is 0.008%.

It was shown according to the invention that using methylene blue at a concentration of 0.02% in the thrombin solution resulted in a reduction of about 50% in thrombin clotting activity after exposure to 6 hours day light. However, when the methylene blue dyed solution was light protected no reduction in thrombin activity was found. In contrast, exposure to 6 hours day light of 0.02% indigo carmine dyed thrombin solution had no effect on the thrombin activity. Moreover, longer exposures of indigo carmine dyed thrombin solution to light (16 hours) did not interfere with thrombin clotting activity. Therefore, for optimal thrombin activity when using methylene blue as the dye protection from light is important.

Thus, in certain embodiments of the invention, the component containing the visualization agent is protected from light. The protection can be achieved by wrapping the recipient with an aluminum foil, by preserving the component comprising the visualization agent in a dark container or recipients or by any other method known in the art. The component comprising the visualization agent can also comprise an agent for the protection from light, such as naturally or synthetic radical scavengers which can substantially prevent or reduce the formation rate of the free radicals without compromising the enzymatic reaction.

Addition of the visualization agent in a concentration which retains from 50 to 100% of the proteolytic enzyme clotting activity can be achieved up to about 0.1% when protected from light, or up to about 0.01% when unprotected from light.

It was found according to the invention that indigo carmine forms aggregates when added into the thrombin solution at concentrations higher than 0.02% when Ca²⁺ is at concentration of 40 mM. A concentration of indigo carmine which is above 0.02% causes aggregates of the indigo carmine resulting in a decrease in coloration compared to the theoretical value if all the IC would have been dissolved. Without being bound to the mechanism, it appears that the aggregates are formed with participation of Ca²⁺ which is essential for thrombin activity and is present in the thrombin component. Thus, it is beneficial that the visualization agent is added to the fibrin glue formulation or kit components at a concentration which allows solubility of the visualization agent without forming aggregates. This concentration with indigo carmine and a concentration of 40 mM calcium is equal or below to 0.02% in the thrombin solution or 0.01% in the fibrin glue. Lower calcium concentration may enable higher concentration of indigo carmine. The term "aggregates" refers to a chunk of material which contains several kinds of solids.

In one embodiment of the invention, the visualization agent in the proteolytic enzyme solution is indigo carmine and the final concentration in the fibrin glue and/or after mixing the kit or formulation components is in the range of about 0.0005 to about 0.01%, from about 0.0025 to about 0.01%, or from about 0.005 to about 0.01% like 0.0075%.

As mentioned above, the visualization agents of the invention are selected from methylene blue and indigo carmine.

The use of dyed fibrin glue is of advantage in surgical environment, for example, when using it for adhesion prevention indications by enabling the surgeon to visualize the FS during application, especially when performing a laparoscopic process. The dyed fibrin sealant can be applied e.g. as spray or by drip as described by Wiseman et al., ("The effect of tranexamic acid in fibrin sealant on adhesion formation in the rat". J Biomed Mater Res B Appl Biomater. 2004;68:222-230).

The fibrin glue kit of the invention can be used in minimal invasive procedures (MIS). The patient can receive local anesthesia or general anesthesia. These procedures can be carried out through small incisions or through a body cavity or anatomical opening. Specialized techniques can be used to visualize the operated area such as, miniature cameras with microscopes, tiny fiber-optic flashlights and high definition monitors. Minimally invasive surgery may result in shorter hospital stays, allows outpatient treatment, can reduce trauma to the body, reduce blood loss, reduces the need for pain medications and reduce morbidity rates as compared to the conventional open surgery. Minimally invasive procedures includes, but are not limited to, laparoscopic, endovascular, laparoscopic splenectomy, laparoscopic umbilical hernia repair, laparoscopic removal of benign ovarian cysts, treatment of herniated lumbar and cervical discs, and the like.

Laparoscopic application of fibrin sealant by spray encompasses the worst-case conditions for targeted spray application. One of the hurdles to overcome is the effect of the laparoscopic fibrin sealant spray application on intra-abdominal pressure (IAP) and on hemodynamics. In a recent publication Druckrey-Fiskaaen et al., ("Laparoscopic spray application of fibrin sealant effects on hemodynamics and spray efficiency at various application pressures and distances". Surg Endosc. 2007;21:1750-1759) reported that fibrin sealants (Quixil) can be safely used in laparoscopic procedures if the following conditions are met: keeping the spray periods short and allowing air to escape from the abdomen. These conditions can minimize the IAP increase. According to their results, a laparoscopic spray application of fibrin sealant should start with an insufflation pressure of 10 mmHg, an application pressure of 2.5 bars, and an application distance of 5 cm with a valve on the trocar left open. This optimization of the spraying conditions opened the way to an efficient application of thin layers of the fibrin sealant in all sorts of laparoscopic applications. However, it hasn't solved the issue of targeting of the relatively thin layer of a transparent gel on a dark, internal bleeding organ. Fibrin sealant application is done very frequently under insufficient lighting when the video camera has accumulated moisture. These harsh conditions call for the spraying of a stained gel that can easily be distinguished from the surrounding tissue.

Fibrin sealant comprising methylene blue at a final concentration of 0.025% was especially useful among other reasons for supplying a clear targeting when sprayed on a dark bleeding organ such as spleen or liver. Furthermore, when sprayed for short periods, generating a thin fibrin glue layer, 0.025% methylene blue allowed for a confident targeting.

It was found that thrombin supplemented with 0.01 and 0.02% indigo carmine solution resulted in superior visibility in laparoscopic application compared to the non-dyed sealant. Thus, fibrin glue containing indigo carmine at a final concentration of 0.005 and 0.01% allows visualization of the sprayed material when spraying the fibrin glue component in dark bleeding or non bleeding organs such as the spleen, liver and uterus.

It was found according to the invention that increasing the concentration of the visualization agent might hinder the clotting (curing) of the fibrin gel, thus the above indicated concentrations have the advantage that allows visibility of the sprayed gel without compromising the stability of the gel layer. Keeping the concentration of methylene blue at 0.01% to 0.05% and of indigo carmine at 0.01 to 0.02% in the thrombin component has been optimized to gain both.

Thus, disclosed herein is the laparoscopic application of a fibrin-glue comprising a dye in a concentration that is enzymatically-permissive and which allows visualization. Also disclosed, the indigo carmine concentration in the thrombin solution is in the range of about 0.01 to about 0.02%, for example the concentration of indigo carmine within the thrombin solution is of about 0.015% or 0.016%.

Yet another object of the invention is accomplished by providing a fibrin sealant kit comprising at least two separate components required to form the fibrin glue, according to the invention, with an enzymatically-permissive concentration of a visualization agent and an application device. The fibrin sealant kit with the applicator device can be used for the prevention and/or reduction of adhesions and/or for promoting blood coagulation or stopping of bleeding, and/or for sealing or filling surfaces.

In another aspect, according to the invention the fibrin glue kits can be used as a hemostatic agent. The term haemostatic agent refers to the ability of the agent to stop the bleeding from an injured blood vessel and/or to contribute to keeping the blood contained within the blood vessel.

In yet another aspect, the fibrin glue kits according to the invention can be used as an anti-adhesive agent. Adhesion formation is an undesired side-effect in which body tissues that are normally separated grow together. This undesirable side-effect may occur after surgical procedures, infection, trauma, or radiation. Typically, anti-adhesive agents refer to agents capable of forming a physical barrier (coating) separating between adjacent tissues at the surgical site and therefore prevent and/or reduce formation of post-operative adhesions.

The fibrin glue kits of the invention can further comprise biologically active molecules such as antibiotics, anti-inflammatory agents, chemotherapy agents, growth factors, anti-cancer drugs analgesics, proteins, hormones, antioxidants and the like. The fibrin glue kits of the invention can be advantageously used as a drug delivery system because the visualization agent allows improved targeting qualities to the application site, for example: improves locating the targeted area, allows controlled release over an extended period and enables delivery of a required concentration which cannot be achieved in an oral delivery.

The term drug delivery system refers to delivery of bioactive molecules which are incorporated into the fibrin glue kit which allow controlled delivery of the molecules in a specific tissue in vivo.

It was found according to the invention that addition of dye within the above mentioned concentration range had no critical effect on the kinetics of the clot formation and on the elasticity and the strength of the clot. Thus keeping these concentration ranges were found to give desirable color intensity and at the same time substantially preserve thrombin clotting activity, and the physical and mechanical characteristics of the glue.

The disclosure of ranges in the description of the invention is easily understood by the skilled person. It means the disclosure of continuous values and figures between the limits of the ranges, including the limiting figures and values. For example, if a range is given of from 0.0025 to 0.1, it is meant at least 0.0025, 0.003, 0.004, 0.005, 0.006, 0.007, 0.008, 0.009, 0.01, 0.02, 0.03, 0.04, 0.05, 0.06, 0.07, 0.0075, 0.08, 0.09, and or 0.1 with all combinations of intermediate sub ranges such as 0.0025 and 0.01, 0.0025-0.02, 0.0025-0.03, 0.0025-0.04, 0.0025-0.05, 0.0025-0.06, 0.0025-0.07, 0.0025-0.08, 0.0025-0.09, 0.0025-0.1 or 0.01-0.02, 0.01-0.03, 0.01-0.04, 0.01-0.05, 0.01-0.06, 0.01-0.07, 0.01-0.08, 0.01-0.09, 0.01-0.1 and so on.

The following examples are illustrative but not limiting.

### Examples

### Example 1: Effect of different dyes on thrombin clotting activity.

The present study was aimed to determine the effect of dye addition to the fibrin glue formulation on thrombin activity. For this purpose, thrombin of a two component fibrin sealant like the one described in US-B-6,121,232 and WO9833533 was formulated with different dyes to final concentrations of 0.01-0.2%. The compatibility of the dyes with thrombin was tested by measuring thrombin clotting activity in the different formulations according to the following modified, European Pharmacopeia Assay (0903/1997), procedure.

Briefly, standard solution of thrombin (4, 6, 8 and 10 IU/ml) or the test sample were incubated for 2 minutes at 30°C. Then 40 µl thrombin solution of each solution were mixed with 160 µl fibrinogen solution (0.1%; Enzyme research; cat No FIB1 2800L) and clotting time was measured. A calibration curve of log clotting times vs. log thrombin concentration was plotted using the standards. Thrombin activity in the different formulations was determined by the clotting time obtained (calculated automatically by a clotting machine, interpolated from the calibration curve and multiplied by the dilution factor).

The following table summarizes the thrombin activity in the different formulations

### (Table 1):

**Table 1: Thrombin activity in the different formulations**

| **Dye** | **Dye concentration within the thrombin component (%)** | **Thrombin Activity (IU/ml)** | **Recovered activity (%)** |
|---|---|---|---|
| **Methylene Blue** | 0 | 1102 | 100 |
| | 0.01 | 1018 | 92 |
| | 0.05 | 1070 | 97 |
| | 0.1 | 991 | 90 |
| | 0.2 | 978 | 89 |
| **Crystal Violet** | 0 | 1057 | 100 |
| | 0.01 | 1125 | 106 |
| | 0.05 | 995 | 94 |
| | 0.1 | 516 | 49 |
| | 0.2 | 234 | 22 |

| | | | |
|---|---|---|---|
| * Crystal Violet was purchased from Sigma (cat No 229288). | | | |

Methylene blue and crystal violet were tested for their effect on thrombin clotting activity at concentration 0.01-0.2%. The findings suggest that methylene blue is more compatible with thrombin than crystal violet e.g., the recovered activity of thrombin with 0.1% methylene blue was 90% vs. 49% activity of a formulation with 0.1% crystal violet. Furthermore, a formulation with 0.2% methylene blue also exhibited high recovered activity compared to crystal violet at the same concentration (89% and 22% recovered activity, respectively).

### Example 2: Effect of different dyes on clotting kinetics.

Human thrombin was mixed with different dyes to final dye concentrations of 0.005-0.2%. The influence of dyes on clotting kinetics was tested using the drop test model. Briefly, measurements of fibrin clotting kinetics were performed on an inclined plane in a device powered by a Nitrogen pressure of 7 X 10⁵ Pa. In each experiment 5 ml of Biological Active Component (BAC) and 5 ml of a thrombin solution 5 folds diluted (final: 200 IU/ml) (in 40 mM CaCl₂) were pumped into a separate syringe. BAC is prepared from concentrated cryoprecipitate after being worked up as disclosed in EP-A-534 178 in which arginine and tranexamic acid are added as described in US-B-6,121,232 and WO9833533). These two solutions were released (about 1/8 of each) simultaneously, and a mixed drop falls onto a slanted surface. The drop leaks down the slope until a clot is formed. The distance traveled by the drops was recorded on a millimetric paper sheet placed on the slanted surface. The distance traveled by the drop was shown to be reversibly proportional to the concentration of thrombin. The migration lengths of the fibrin sealant with the different formulations are listed below in Table 2.

**Table 2: Migration length of the fibrin sealant in the different formulations**

| **Dye** | **Dye concentration within the thrombin component (%)** | **Migration length (cm)** | **±SD (N) (cm)** |
|---|---|---|---|
| **Methylene Blue** | 0 | 9.4 | 0.95 (8) |
| | 0.1 | 10.7 | 2.7 (8) |
| | 0.2 | 14.3 | 0.3 (8) |
| **Crystal Violet** | 0.005 | 12.1 | 1.9 (8) |
| | 0.01 | 23.9 | 1.0 (8) |
| | 0.05 | >25 | * ND (8) |
| **Riboflavin** | 0.005 | 11.5 | 1.7 (8) |
| | 0.01 | 13.3 | 1.6 (8) |
| | 0.02 | 22.9 | 2.4 (8) |
| **Methyl Orange** | 0.005 | 8.26 | 1.6 (8) |
| | 0.01 | 7.6 | 1.2 (8) |
| **Bromothymol Blue** | 0.01 | 9.7 | 1.1 (8) |
| | 0.02 | 11.9 | 0.9 (8) |

| | | | |
|---|---|---|---|
| * Riboflavin was purchased from Merck KGaA (cat No 500257). * Methyl Orange Sodium Salt was purchased from J.T. Baker (cat No 1145). * Bromothymol Blue was purchased from BAKER ANALYZED. *ND- not determined. | | | |

| **Dye** | ***Dilution** | **Migration length (cm)** | **±SD (N) (cm)** |
|---|---|---|---|
| **Methyl Red** | 01:51 | 10.2 | 2.05 (8) |
| | 01:26 | 12.4 | 0.8 (8) |

| | | | |
|---|---|---|---|
| * Methyl red was purchased from J.T. Baker (cat No 5926-04) and diluted as specified above. | | | |

These results confirm the above results showing that methylene blue at a concentration range of 0.1-0.2% mostly retained the thrombin clotting activity as compared to the non-dyed formulation.

On the other hand, it is apparent that in the drop test model crystal violet at a concentration range of 0.01-0.05% strongly interfered with the clotting activity whereas in the thrombin clotting activity assay exemplified above (Example 1) crystal violet in the same concentration range did not cause interference in thrombin activity (duplication of the traveled distance in the drop test model vs. 106 and 94% Thrombin activity recovery in the direct thrombin activity assay). As was shown in this assay, 0.005-0.01% riboflavin, 0.005-0.01% methyl orange, 1:51-1:26 diluted methyl red and 0.01-0.02% bromothymol blue did not dramatically interfere with clotting kinetics.

### Example 3: Stability of the clotting activity of fibrin-glue with the visualization agent (dye) after freeze and thaw (F&T).

Human thrombin was formulated with methylene blue, crystal violet, bromothymol blue or riboflavin at final concentrations of 0.005-0.1%. The different formulations were fast frozen to -35°C and then thawed. The effect of the freeze and thaw procedure on thrombin clotting activity was evaluated using the drop test model (migration time assay as in Example 2). The results are summarized in Table 3.

**Table 3: Effect of the freeze and thaw procedure on the stability of the glue**

| **Dye** | **Dye concentration within the thrombin component (%)** | **Migration length (cm)** | **±SD (n) (cm)** |
|---|---|---|---|
| **Methylene Blue** | 0.1 | 10.7 | 2.7 (8) |
| | 0.1 (F&T) | 8.4 | 2.1 (8) |
| **Crystal Violet** | 0.005 | 12.1 | 1.9 (8) |
| | 0.005 (F&T) | 16.3 | 1.0 (9) |
| **Bromothymol Blue** | 0.02 | 11.9 | 0.9 (9) |
| | 0.02 (F&T) | >25 | * NA(9) |
| **Riboflavin** | 0.005 | 11.5 | 1.7 (9) |
| | 0.005 (F&T) | 19.8 | 2 (9) |

| | | | |
|---|---|---|---|
| *NA- Not Available | | | |

The clotting activity of the fibrin glue formulated with methylene blue did not change significantly as a result of freezing and thawing procedure.

Thus, the experiment shows that clotting activity of the fibrin glue formulation with methylene blue is stable even though the formula was frozen and thawed.

### Example 4: Solubility of indigo carmine in thrombin solution.

The present example was aimed to determine the maximal solubility of indigo carmine in the thrombin component of the fibrin sealant. Thrombin final container (Omrix, 1,000 IU/ml, 5 ml) was mixed with 1% indigo carmine (dissolved in purified water) to a final concentration of 0.2, 0.21, 0.22, 0.25 or 0.3 mg/ml. The prepared solutions were mixed on a roller for 30 min at room temperature and the solubility limit of the dye was determined by visual inspection.

It has been shown that complete dissolution of the sample was obtained only at a concentration of 0.2 mg/ml. Higher concentrations of indigo carmine dyed thrombin solution (i.e. 0.21, 0.22, 0.25 and 0.3 mg/ml) exceeded the solubility limit and resulted in aggregate formation. It appears that the aggregates are formed with participation of Ca²⁺ present in the thrombin component. These data suggest that the limit solubility of the indigo carmine within the thrombin solution tested (40 mM Ca²⁺) is of about 0.02%.

Also, it was found that storage at 2-8°C of the 0.02% indigo carmine dyed thrombin solutions for about 30 min resulted in sedimentation. Incubation of the refrigerated 0.02% indigo carmine dyed thrombin solutions at room temperature (after over night incubation at 2-8°C) lead to re-dissolution of the particulate sample as was observed by visual inspection.

### Example 5: The effect of indigo carmine and methylene blue on thrombin activity.

In the adhesion prevention indications the dye may be added to the thrombin component prior to mixing it with BAC. The present experiment was carried out to assess the effect of visualization agent addition to thrombin solution on thrombin clotting activity. Two dyes were evaluated: Indigo Carmine (IC) and Methylene Blue (MB). For this purpose, the thrombin final container (Omrix, 1,000 IU/ml) was mixed with either MB or IC to a final concentration of 0.02%. The effect of the dyes on thrombin activity with or without exposure to day light was assessed. The thrombin clotting activity was measured as indicated above in Example 1. A calibration curve (log clotting time vs. log thrombin concentration) was prepared by mixing thrombin standards with a 0.1% fibrinogen solution. The samples were mixed with the same fibrinogen solution and the thrombin activity was calculated from the calibration curve. Thrombin final container (Omrix), indigo carmine (Amresco code cat No 9827-25g), methylene blue (Spectrum cat No ME141-25g-USP), spectrophotometer and a clotting machine were used. For the preparation of 1% IC and 1% MB solutions 0.04 g of either IC or MB were added into 4 ml of purified water.

Three 5 ml vials of thrombin final container were used in this experiment:
1. The first thrombin vial was dyed with IC by adding 0.1 ml of 1% IC solution into 4.9 ml of thrombin to achieve IC final concentration of 0.2 mg/ml.
2. The second thrombin vial was dyed with MB by adding 0.1 ml of 1% MB solution into 4.9 ml of thrombin to achieve MB final concentration of 0.2 mg/ml.
3. The third thrombin vial was left untreated.

The three 5 ml vials were divided each to 2 aliquots of 2.5 ml in transparent vials; subsequently one aliquot from each group was covered with an aluminum foil. All the samples were incubated at room temperature exposed to day light.

Thrombin activity and the color intensity (OD of IC at 610 nm and of MB at 663 nm) were measured at T0 and after exposure to day light for 6 hours. This experiment was repeated twice to yield duplicates.

The results obtained demonstrated that in the covered samples thrombin activity and OD values were not influenced by the exposure to light, regardless of IC or MB presence (Tables 4&5). The exposure of the IC dyed thrombin solution, as that of the un-dyed thrombin solution, to day light had no effect on the thrombin activity during the incubation period. However when thrombin solution was dyed with MB and incubated uncovered exposed to day light, a marked reduction of about 50% in thrombin activity was found after incubation for 6 hours. The OD of the MB and IC was left unchanged during the incubation period indicating that the color intensity remained unaltered during day light exposure.

**Table 4: The effect of indigo carmine and methylene blue on thrombin activity**

| **Sample** | **Thrombin activity IU/ml *** | |
|---|---|---|
| | **T0** | **6 hours** |
| **L51 T60** | 1155±81 | 1047±23 |
| **L51T60 + Aluminum foil** | | 1001±9 |
| **L51T60 + IC** | 1056±0 | 1117±67 |
| **L51T60 + IC + Aluminum foil** | | 1124±36 |
| **L51T60 + MB** | 1091±30 | 514±7 ** |
| **L51T60 + MB + Aluminum foil** | | 1070±81 |

| | | |
|---|---|---|
| * The results are an average of two independent replicates. ** This result is estimation since it was out of the assay calibration curve. The final result can be accurately stated as being <564 IU/ml. | | |

**Table 5: The influence of light exposure on OD values of dyed thrombin solutions**

| **Sample** | **Exposure to day light *** | |
|---|---|---|
| | **T0** | **6 hours** |
| | **OD at 610 nm** | |
| **L51T60 + IC** | 0.359±0.002 | 0.354±0.001 |
| **L51T60 + IC + Aluminum foil** | | 0.357±0.004 |

| | **OD at 663 nm** | |
|---|---|---|
| **L51T60** + **MB** | 0.518±0.007 | 0.522±0.001 |
| **L51T60 + MB + Aluminum foil** | | 0.527±0.001 |

| | | |
|---|---|---|
| * The results are an average of two independent replicates. | | |

The results clearly demonstrated that using indigo carmine at final concentration of 0.02% in the thrombin solution did not affect thrombin activity regardless of exposure to day light for up to 6 hours. In contrast, when the same concentration of methylene blue was used as the dye, a marked reduction of thrombin activity was observed after equal exposure to day light. However, when methylene blue dyed solution was light protected (covered with aluminum foil) no reduction in thrombin activity was found throughout the study period. Therefore, the results indicate that there is no need to protect thrombin from light when using indigo carmine as the dye for thrombin, whereas protection from light is important with methylene blue.

### Example 6: The effect of indigo carmine on thrombin activity following a prolonged incubation period.

The above example shows that addition of indigo carmine to the thrombin component at a final concentration of 0.02% had no effect on thrombin clotting activity even when exposed to 6 hours day light. The present example was aimed to determine the effect of prolong incubation period of the indigo carmine dyed thrombin solution on thrombin clotting activity. Both day light exposed and un-exposed samples were examined.

For this purpose, 0.4% indigo carmine solution (dissolved in purified water) was mixed with 5 ml thrombin solution (1:26) as to achieve a final concentration of 0.15 mg/ml. The mixed solutions were incubated at room temperature for 27 hours (16 hours day light) in clear or amber vials.

Samples (40 µl) were taken out from the vials at the following time points: 0, 4, 22, and 27 hours after the initiation of the experiment and thrombin activity was determined according to the method describe above (Example 1). The measurements were carried out in duplicates.

Table 6 summarizes the effect of prolonged incubation of the indigo carmine dyed thrombin solution on thrombin activity with or without exposure to day light. The results obtained are also expressed as a fold decrease in thrombin clotting activity as compared to the activity of the sample at T0 (100%; Fig. 1A and B for two different batches of thrombin). IC- indigo carmine.

**Table 6: The effect of indigo carmine on thrombin activity after prolonged incubation of both day light exposed and unexposed samples**

| **Thrombin vial** | **Sample** | **Thrombin activity (IU/ml)** | | | |
|---|---|---|---|---|---|
| | | **Exposure time (hours)** | | | |
| | | **0** | **4** | **22** | **27** |
| **L51T60K** | Thrombin | 900±12 | 912±6 | 809±11 | 732±8 |
| | Thrombin + indigo carmine; clear vial | 921±6 | 926±25 | 816±10 | 785±23 |
| | Thrombin + indigo carmine; amber vial | 903±5 | 917±37 | 816±20 | 747±13 |
| **M03T08K** | Thrombin | 990±14 | 966±20 | 806±34 | 782±0 |
| | Thrombin + indigo carmine; clear vial | 971±14 | 966±20 | 875±12 | 819±4 |
| | Thrombin + indigo carmine; amber vial | 896±6 | 962±13 | 852±0 | 827±25 |

The results indicate that addition of indigo carmine at a final concentration of 0.015% in the thrombin solution does not interfere with thrombin clotting activity even when exposed to 16 hours day light (85 and 84% as compared to 81 and 78% recovered activity for indigo carmine dyed thrombin solution in a clear vial and non-dyed thrombin solution, respectively). These results verify that light protection is not required when using indigo carmine as the dye for thrombin solution.

### Example 7: The effect of freezing and thawing of the indigo carmine solution on thrombin activity.

The stability of the clotting activity of the thrombin solution was evaluated following supplementation with indigo carmine solution which was subjected to either one or five cycles of freezing and thawing. The thawed indigo carmine solutions (0.4% dissolved in purified water) were diluted 1:26 in 5 ml thrombin component (Omrix) obtaining an indigo carmine dyed thrombin solution at a final concentration of 0.15 mg/ml. Non-dyed thrombin solution was used as control. Thrombin clotting activity was measured as indicated above (Example 1). Each measurement was carried out in duplicates.

**Table 7: Effect of freezing and thawing of the indigo carmine solution on thrombin clotting activity**

| **Thrombin vial** | **Thrombin activity (IU/ml)** | | |
|---|---|---|---|
| | Thrombin | Thrombin + Indigo carmine* | Thrombin + Indigo carmine +F&T** |
| **L51T06K** | 819±56 | 897±33 | 912±48 |
| **M03T08K** | 932±5 | 1002±47 | 941±30 |

| | | | |
|---|---|---|---|
| The indigo carmine solution was subjected to either one (*) or five cycles (**) of freezing and thawing. | | | |

The results show that thrombin activity is substantially retained in all experimental groups, regardless of multiple freezing and thawing cycles of the indigo carmine solution prior to the addition to the thrombin component.

### Example 8: The effect of indigo carmine on clotting time when added to the BAC component.

The visualization agent can be added to the BAC prior to mixing it with thrombin component. Thus, this example illustrates the effect of indigo carmine on clotting time when added to the BAC.

0.4% indigo carmine solution (dissolved in purified water) was diluted 1:26 into 5 ml BAC component to a final concentration of 0.15 mg/ml. Non-dyed BAC was used a control.

The clotting time was assessed according to the modified European pharmacopeia assay (0903/1997), which is based on the Clauss method. Briefly, a calibration curve was prepared by diluting 1% Fibrinogen solution [Enzyme Research; cat No FIB1 2800L dissolved in Owren-Koller buffer (Diagnostica Stago; cat No 00360)] to final concentrations of 38.46, 25, 12.5, and 8.3 mg/100 ml. The dilutions were carried out in dilution buffer containing 1 % Bovine Albumin in Owren-Koller buffer. Then, about 0.03 g dyed or non-dyed BAC samples were diluted 1:300 in dilution buffer to obtain a final fibrinogen concentration of about 0.2 mg/ml. Clotting was achieved by mixing 100 µl of the above diluted BAC samples with 100 µl Fibri-Prest Automate 2 (Diagnostica Stago; cat No 00316). Clotting time was measured 1 and 3 hours following incubation at room temperature using a clotting machine (ST2 or ST4 Diagnostica Stago).

Based on the obtained clotting time, the sample's fibrinogen concentration is interpolated from the calibration curve. The output contains the clotting time and the calculated fibrinogen concentration. The clotting time in the different samples are listed in Table 8 below.

**Table 8: Effect of indigo carmine solution on clotting time when added to the BAC component**

| **Sample** | **Clotting Time (seconds)*** | | |
|---|---|---|---|
| | **Incubation Time (hours)** | | |
| | **0** | **1** | **3** |
| **K49B252** | 13.8±0.8 | 13.8±0.7 | 13.5±1.0 |
| **K49B252 + indigo carmine** | 13.5±0.2 | 13.5±0.5 | 14.0±0.6 |

| | | | |
|---|---|---|---|
| * Each sample was tested in duplicates and each duplicate was tested twice. The data presented are the average of all 4 measurements obtained for each sample at each time point. | | | |

According to the results obtained, it is apparent that indigo carmine did not change the clotting time at all time points (i.e. 0, 1 and 3 hours). These finding suggests that indigo carmine can be added to the BAC component without altering the clotting activity and the time needed to generate a clot.

### Example 9: The effect of indigo carmine addition on the mechanical properties of the formed clot.

The following example was aimed to determine whether addition of indigo carmine to the fibrin glue formulation affects the elastic modulus of the generated clot.

The mechanical properties of the fibrin clot were measured by an elongation test using a LF Plus model (Lloyd instrument) apparatus. This instrument is a motor-driven tension and compression tester designed for testing the resilience, yield points and breaking strengths of various products and materials. Two conical shaped casts, which are pre-coated with vaseline solution (10% in Hexane) to prevent adhesion to the casts, were placed one on top of the other. The casts were filled with fibrin glue as follows:

Dyed (at a final concentration of 0.15 mg/ml indigo carmine obtained as described above) or non-dyed thrombin standard solution (Omrix, In house STD 139 IU/ml), were diluted in 40 mM CaCl₂ to achieve a thrombin activity of 8 IU/ml. BAC (Omrix) was applied with an equal volume of the diluted thrombin samples into the casts at a total volume of 0.7 ml using a dual syringe module. The prepared clots were incubated at 37°C for 30 minutes to allow full polymerization of the glue. Then, the casts were mounted onto the LF Plus apparatus and mechanically pulled apart.

The strength of the clot was measured by plotting the force exerted (y) versus the distance traveled (x) by the upper cast prior to the breaking point of the clot. The data were collected and processed using the NexyGen Plus software (Ametek Company) which supports the LF Plus apparatus. The processed data was used to generate a stress-strain curve and to calculate the Young's Modulus also known as the Modulus of Elasticity which is represented by the slope of the stress-strain curve. The results are expressed in kPa. Table 9 summarizes the results of these studies:

**Table 9: Effect of indigo carmine solution on the clot's elasticity**

| **BAC Sample** | **Young's Modulus (kPa)** | |
|---|---|---|
| | **Undyed Thrombin** | **Thrombin + indigo carmine** |
| **J26B162** | 12.7 ± 0.7 | 13.5 ± 1.3 |
| **K49B252** | 16.1 ± 0.8 | 16.8 ± 0.4 |
| **K51B262** | 14.1 ± 1.1 | 13.5 ± 0.7 |

Elasticity measurements of the clot show that, addition of indigo carmine at a final concentration of 0.0075% in the generated clot has no effect on the stiffness of the clot. These results demonstrate that addition of indigo carmine into the fibrin glue formulation does not alter the clot elastic modulus consequently resulting in fibrin glue with superior mechanical properties.

### Example 10: The effect of indigo carmine addition on the clotting kinetics and the stiffness of the formed clot

The following example was to asses the affect of indigo carmine on clot formation and stiffness. This was carried out using the Thromboelastograph (TEG), which evaluates the parameters of coagulation in blood and blood products.

The following parameters were evaluated using a hemostasis analyzer (TEG-5000, Haemoscope Corporation): the R-time, K-time, Angle (α), Maximum Amplitude (MA) G, and E.

Reaction time (R) - The time required from the sample placement in the analyzer until the initial fibrin clot formation.

Time (K) - a measure of the time until a certain level of clot strength is obtained. The time is measured from R until a fixed level of clot firmness is developed. K represents the kinetics of clot formation.

Angle (α, grade) - Measures the rapidity of fibrin build up and cross linking. This measure reflects the clotting kinetics.

Maximum amplitude (MA) - represents the maximal strength or stiffness of the developed fibrin clot.

G (shear elastic modulus strength) is a measure of clot strength.

E is a normalized G parameter and is referred to as elasticity constant.

The assay procedure was as follows: BAC (Omrix) was diluted 1:9 in Owren-Koller buffer (Diagnostica Stago cat No 00360) Thrombin (Omrix In-House standard, 139 IU/ml) was diluted in 40 mM CaCl₂ solution to achieve thrombin activity of 10 IU/ml. 0.4% indigo carmine solution (prepared in purified water) was added to each of the diluted fibrin glue components as to achieve a final concentration of 0.075 mg/ml. The diluted BAC solution (340 µl) was mixed with the diluted thrombin solution (20 µl) inside a designated testing cup. The cup was then placed into the TEG analyzer and the developed clots parameters were collected. The obtained clots parameters are presented below (Table 10). Each test was performed in duplicates.

**Table 10: Evaluation of IC influence on clotting kinetics and clot stiffness using Thromboelastography**

| **Sample** | **R (min)** | **K (min)** | **Angel α (grade)** | **MA (mm)** | **G (Kd/sc)** | **E (d/sc)** |
|---|---|---|---|---|---|---|
| **J26B162** | 2.2±0.1 | 2.5±0.4 | 54.9±7.1 | 42.6±1.1 | 3.7±0.1 | 74.2±3.1 |
| **J26B162 + indigo carmine** | 2.0±0.2 | 2.3±0.4 | 60.1±5.5 | 45.1±3.7 | 4.2±0.6 | 82.4±12. 3 |
| **K47B240** | 1.9±0.3 | 2.1±0.1 | 62.2±0.7 | 44.3±0.21 | 4.0±0.1 | 79.4±0.6 |
| **K47B240 + indigo carmine** | 1.7±0.2 | 2.4±0.8 | 60.7±8 | 43.6±2.8 | 3.9±0.4 | 77.3±8.7 |

Measured Thrombelastograph parameters were not significantly changed as a result of indigo carmine addition to the fibrin glue formulation at a final concentration of 0.0075% in the generated clot.

These results provide evidence suggesting that the clotting kinetics, the clot stiffness and the maximal strength of the developed clot are not compromised as a result of visualization agent addition to the fibrin glue composition.

### Example 11: The effect of indigo carmine on clot longevity.

The purpose of the following example was to test the effect of adding a visualization agent to the fibrin glue on the clot longevity in-vivo. The dye chosen as a substance coloring was indigo carmine. Non-dyed fibrin clot served as reference.

Clot longevity was determined in Sprague-Dawley rats weighing 300-400g and over the age of 9 months. Each testing group included 15 animals. Allocation to treatment groups was done during the acclimatization period, using a random stratified procedure.

Before and after surgery, the animals were housed in the animal room in an air-conditioned room, in a temperature range of 22±4°C, relative humidity of 30-70% and under an artificial lighting cycle (12 hours artificial light: 12 hours dark). The animals were put in cages (1 or 2 animals in each polycarbonate cage; 42x26x18cm) with free access to food and to sterilized tap water. The animals were examined daily and weighed at the beginning and at the end of the study.

Prior to surgery animals were anesthetized with a 40-80 mg/kg IM injection of a mixture of 85/15 Ketamine HCl 100 mg/ml and Xylazine HCl 20 mg/ml.

The abdominal wall defect model was used as described by Wiseman et al., ("The effect of tranexamic acid in fibrin sealant on adhesion formation in the rat". J Biomed Mater Res B Appl Biomater. 2004;68:222-230). Briefly, the rats were shaved and a 6 cm incision marked on the skin overlaying the linea on the ventral midline. With the muscle wall exposed, a 5 cm incision in the muscle was made along the linea all through the peritoneal cavity. The right abdominal wall was reflected. A 2 cm x 1 cm of the peritoneum was removed. The medial edge of this defect was located 1 cm lateral from the midline incision and parallel to it. The abdominal wall defect was exposed to air for 10 minutes to monitor any bleeding.

The wounds were sprayed with the fibrin glue preparations which included BAC as in Example 2 (0.5 ml) and thrombin as in Example 4 (0.5 ml) (1 ml of total glue). In the test sample group the thrombin component was supplemented with indigo carmine at a concentration of 0.16 mg/ml (0.016%). The resulting fibrin clot contained Indigo Carmine at a concentration of 0.08 mg/ml (0.008%). The midline incision and the skin were closed with a running 2-0 Dexon bi-color suture. The animals in the two groups were sacrificed 1, 3, 5, 7 and 12 days after the initiation of the experiment, 3 animals of each group, at each time point.

At the end of the predefined time intervals, animals were euthanized using intraperitoneal injection of 0.7 ml Pental 200 mg/ml per rat. A V-shape incision was made exposing the abdominal wall. The remains of the clot were removed from the rat abdomen, extracted, weighed, dissolved in clot dissolving solution and tested for protein as described below.

Each clot was washed with saline, placed into a test tube containing clot solubilising solution (0.5-5 ml depending on the clot's size; 7M urea and 0.2M NaOH in a PBS-sodium chloride 0.9% buffer mixed at a ratio of 1:2. The test tube was left to stand at room temperature until the clot has been completely dissolved, as judged by visual inspection. Protein concentration in the remaining clot of each sample following clot solubilization was quantitatively determined by the following procedure. 0.1 ml solubilised clot solution was diluted in PuW (1:10) and read at 280 nm. The measurements were carried out in 1 ml cuvettes. The clot protein was determined after reduction of light scattering at 320 nm and interpolation from a known internal standard. The actual volume of the clot solubilizing solution used to dissolve the clot remains was taken into account for calculation of the protein amount. The Clot weight and the Clottable protein amount of the dyed and non-dyed fibrin glue formulations are presented in Table 11 and 12, respectively.

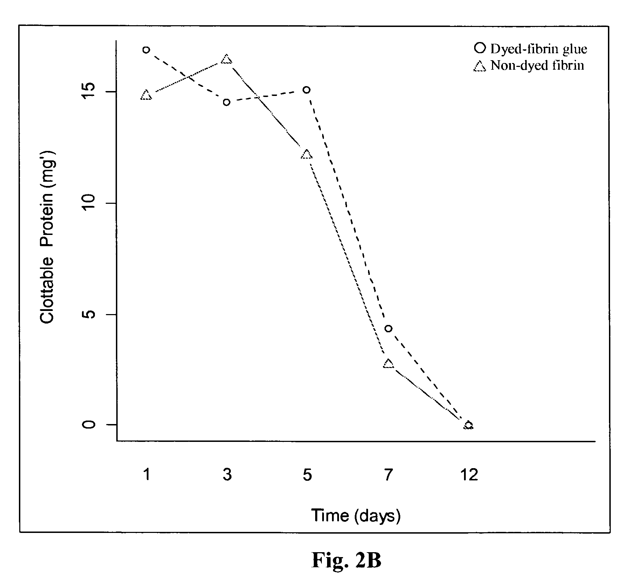

The mean clot weight and the mean clottable protein amount in the various time points are presented in Fig. 2A and B, respectively

**Table 11: Clot Weight and clottable protein amount in the different time points of the indigo carmine dyed fibrin glue**

| **Animal No.** | **Time** (days) | **Clot weight** (g) | **Clottable protein** (mg) |
|---|---|---|---|
| T1-1 | 1 | 0.226 | 12.5 |
| T1-2 | | 0.161 | 19.9 |
| T1-3 | | 0.219 | 18.1 |
| **Average T1** | | **0.202±0.036** | **16.8±3.9** |
| T3-1 | 3 | 0.1521 | 12.2 |
| T3-2 | | 0.2035 | 14 |
| T3-3 | | 0.2321 | 17.4 |
| **Average T3** | | **0.196±0.041** | **14.6±2.6** |
| T5-1 | 5 | 0.1135 | 12.5 |
| T5-2 | | 0.1702 | 17.1 |
| T5-3 | | 0.1298 | 15.6 |
| **Average T5** | | **0.138±0.029** | **15.1±2.3** |
| T7-1 | 7 | 0.0482 | 3.9 |
| T7-2 | | 0.0209 | 1.1 |
| T7-3 | | 0.1023 | 8.1 |
| **Average T7** | | **0.057±0.041** | **4.3±3.5** |
| T12-1 | 12 | none | none |
| T12-2 | | none | none |
| T12-3 | | none | none |
| **Average T12** | | **NA** | **NA** |

**Table 12: Clot Weight and clottable protein amount in the different time points of the non-dyed fibrin glue**

| **Animal No**. | **Time** (days) | **Clot weight** (g) | **Clottable protein** (mg) |
|---|---|---|---|
| T1-1 | 1 | 0.201 | 13.2 |
| T1-2 | | 0.301 | 20.1 |
| T1-3 | | 0.143 | 11.1 |
| **Average 1** | | **0.215±0.080** | **14.8±4.7** |
| T3-1 | 3 | 0.2431 | 20 |
| T3-2 | | 0.1468 | 14.1 |
| T3-3 | | 0.1651 | 15.1 |
| **Average 3** | | **0.185±0.051** | **16.4±3.2** |
| T5-1 | 5 | 0.1452 | 15.4 |
| T5-2 | | 0.0891 | 8.4 |
| T5-3 | | 0.1346 | 12.7 |
| **Average 5** | | **0.123±0.030** | **12.2±3.5** |
| T7-1 | 7 | 0.0183 | 2 |
| T7-2 | | 0.0404 | 4.4 |
| T7-3 | | 0.0215 | 1.9 |
| **Average 7** | | **0.027±0.012** | **2.8±1.4** |
| T12-1 | 12 | none | none |
| T12-2 | | none | none |
| T12-3 | | none | none |
| **Average 12** | | **NA** | **NA** |

The results indicate that from day 3 the clot weight declines with time in both tested groups, i.e. dyed and un-dyed fibrin glue (Fig. 2A). A reduction in the clottable protein amount was apparent since the 5th day in both groups (Fig. 2B). No significant differences were found between the groups regarding the clot's weight and the clottable protein amount. These results demonstrate that addition of indigo carmine at a final concentration of 0.008% in the generated glue does not alter the fibrinolysis rate and the longevity of the clot in an in vivo setting.

Additionally, it should be noted that following the first day no dye was apparent in the clot as was determined spectrophotometrically or by visual inspection.

### Example 12: Laparoscopic visibility of fibrin glue containing different concentrations of methylene blue and indigo carmine in intraperitoneal cavity.

The objective of these experiments was to study the effect of different concentrations of the tested visualization agents on fibrin glue (as described in US-B-6,121,232 and WO9833533) visibility in laparoscopy procedure during spraying on non-bleeding or bleeding organs. The visibility of the tested product was determined using a pig interperitoneal laparoscopic model with or without induction of oozing on the organ's surface. The visibility of two coloring substances was tested: methylene blue and indigo carmine. Adult female domestic crossbreed swine (n = 1) weighing about 50 kg and under the age of 2 years were housed in an authorized facility according to the current ethical requirements.

Three laparoscopic ports were placed in the abdomen of the pig. The fibrin glue preparation was applied to the target site by spraying. The testing manipulation procedure was repeated for each of the tested material concentrations and the control (undyed) trial. The fibrin glue vials were thawed shortly prior to use and the dyes added to the Thrombin vials.

A new application device was used for each concentration. Each application site on the organ of choice was distant enough from previous application sites to allow distinguishing between the applications. The operation and the application site were recorded before and after the application of the fibrin glue. The fibrin glue application was recorded from different angles and different zoom settings.

Surgical application on bleeding site: Oozing of the surface of the organ of choice was induced by rubbing the organ's surface with sandpaper inserted with the laparoscope clips to the abdomen.

### Application of fibrin glue supplemented with methylene blue

Fibrin glue undyed or supplemented with three different methylene blue concentrations was applied to bleeding spleen surface. The fibrin glue included: 1) BAC as in Example 2 (5 ml). 2) Thrombin (1000 IU/ml; 5ml). Thrombin was supplemented with the following methylene blue concentrations: 50, 100 or 500 ppm (concentrations of 0.005, 0.01, and 0.05%, respectively).

Three evaluators were appointed by the laboratory director for independent evaluation of the visibility of the fibrin glue with the different concentrations of the methylene blue. The evaluator took into account two parameters: 1) Color contrast between spleen surface and applied material. 2) Color contrast between blood and applied material. The evaluators have independently voted for a concentration of 500 ppm of methylene blue in the thrombin vials of the glue as the concentration which provides the highest visibility to the fibrin glue. All MB concentrations tested showed superior visibility compared to the undyed fibrin glue.

### Application of fibrin glue supplemented with indigo carmine

Two indigo carmine concentrations were applied and compared to non-dyed product. The visibility test was carried out on two dark organs (spleen and liver) on both bleeding and non bleeding sites. In addition, the dyed fibrin glue was tested on a light organ (uterus) only on a bleeding site and only with dye (due to the limited size of the organ).

The fibrin glue included BAC and Thrombin as indicated above. The thrombin component was supplemented with the following indigo carmine concentrations: 0.2 mg/ml and 0.1 mg/ml (0.02% and 0.01%, respectively). 1 ml from each component was sprayed in each application.

The different applications were estimated by seven evaluators that scored the visibility of the sprayed product in each case. The visibility was graded based on the contrast between the applied substance and the organ surface or pool of blood (the visibility was graded from 1 to 10, when 1 represented low visibility and 10- high visibility).

All evaluators agreed that fibrin glue containing indigo carmine was superior to the non-dyed fibrin glue. The results suggested that both 0.01 and 0.02% indigo carmine dyed thrombin solutions enabled clear visualization of the sprayed material even under conditions of spraying on dark organs with mild bleeding. When the indigo carmine was applied to the bleeding spleen surface, where a relatively higher amount of blood diluted the product, fibrin glue containing 0.02 % of indigo carmine was superior in supplying a clear targeting.

It should be noted that additional results showed that increasing the concentration of the visualization agent (e.g. methylene blue and indigo carmine) might hinder the clotting process of the fibrin gel. Thus, the above indicated concentrations are a compromise between the visibility of the sprayed gel and the stability of the gel layer. Keeping these concentrations has been optimized to gain both.

### Example 13: Efficacy of dyed-fibrin glue formulation vs. undyed formulation in reducing post-surgical adhesions.

The fibrin glue formulation of the invention can be used as an anti-adhesive agent. The following example illustrates the efficacy of dyed fibrin glue formulation in reducing post-surgical adhesions.

The indigo carmine solution (Indigotindisulfonate sodium, Amresco, Solon, Ohio) was supplied as a sterile 1% w/v solution in amber vial. Immediately prior to use, 0.1 ml was withdrawn from the vial and mixed with the Thrombin solution (5 ml) for 5 minutes to achieve a final concentration of 0.02% dye (resulting in fibrin glue containing a final concentration of 0.01% dye). Non dyed fibrin glue formulation was used as a reference material.

The evaluation was carried out in a rabbit uterine horn abrasion model, in the presence of bleeding.

Female New Zealand White rabbits (*Oryctolagus cuniculus*) weighing between 2.7-3.3 kg were used.

Animals were acclimated for a minimum of 5 days prior to initiation of the study, and monitored by experienced animal care personnel daily.

Animals were individually housed in stainless steel cages. The room environment was maintained at approximately 20°C with 30-70% relative humidity and a light/dark cycle of 12 hours/12 hours.

Rabbit chow Harlan Teklad 15% Rabbit Diet #8630 (Harlan Teklad, Indianapolis, IN) and tap water were provided *ad libitum* to the animals for the duration of the study. Filtered city water was delivered through an Edstrom Automatic Watering System. The study was performed in accordance with the NIH guidelines as described in the Guide for the Care and Use of Laboratory Animals, National Academy Press, 1996.

### Preparation and Recovery

Animals were weighed on the day of surgery. Anesthesia was induced and maintained by inhalation of isoflurane (5% and 3.5% concentration, respectively). Depilation of the surgical site was accomplished with an electric animal clipper. The area was vacuumed to remove hair clippings and debris, and then rinsed with alcohol. The entire area was scrubbed with Chloroxylenol 3% and left for 5 minutes before removing with 70% Isopropyl alcohol and repeating. The surgical site was cleansed again with 70% isopropyl alcohol. A sterile incise drape was applied to the prepared area.

Three doses of buprenorphine (Buprenex) (0.03 mg/kg, 0.3mg/ml x 0.3ml) were given by subcutaneous injection, one on the morning of surgery, one six to eight hours later and one the following morning. Animals were allowed to recover completely in an incubator prior to returning them to their cages. Thereafter, they were maintained with food and water ad libitum, and observed daily. The incision line was inspected daily for signs of dehiscence and bleeding.

### Rabbit Uterine Horn Abrasion Model

The rabbit uterine horn model was conducted essentially as described by Wiseman et al., ("Effect of thrombin-induced hemostasis on the efficacy of an absorbable adhesion barrier". J Reprod Med. 1992;37:766-770). Briefly, after anesthesia and preparation for sterile surgery a midline incision was made through the skin and the abdominal wall. Both uterine horns were located and exteriorized. Using a French Catheter Scale, the diameter of each uterine horn was measured and recorded. Only those rabbits with uterine horns measuring size 10-16 inclusive on the French scale were entered into this protocol.

Using a number 10 scalpel blade, 5 cm lengths of each uterine horn, along the entire horn length, approximately 1cm from the uterine bifurcation, were scraped, 40 times per side, until punctate bleeding. For the "Bleeding" variation four small vessels in the mesouterine arcade were nicked about 5 mm from the uterus to produce bleeding. After abrasion procedures were completed, the group assignment was revealed to the surgeon. 13 animals received fibrin glue without dye, 11 received fibrin glue supplemented with indigo carmine and five animals served as controls (surgical procedures performed, but no test material was applied). Allocation to the testing groups was done randomly by lottery. Test materials were applied to the uterine horn.

### Application of Test Materials

Between 4.5 ml and 10 ml total volume of dyed (0.02% dye in the thrombin solution as described above) or non dyed fibrin glue were applied to each animal randomized to receive those treatments. After curing (about 120 seconds) the horns were flipped over permit application to the other side. Organs were then replaced anatomically and the incision was closed. Abdominal incisions were closed using a continuous Vicryl 4-0 suture. Fascia was closed loosely with 4-0 Vicryl and the skin closed with undyed 4-0 Vicryl (cutting needle) using a subcuticular suturing method.

### Evaluation

At 13 or 14 days after surgery, animals were euthanized by intravenous injection of sodium pentobarbital (120 mg/ml; 1 ml/kg). Body weights of the animals were recorded. The abdomen was opened and the surgical site was inspected by a blinded observer.

The following parameters were evaluated:
**Extent of adhesions** - The % of the total horn length involved with adhesions expressed as the % of the length of the uterus.
**Tenacity (Severity) of Adhesions** - Adhesions were graded as 0 (absent), 1.0 (filmy adhesions) and 2.0 (tenacious, requiring sharp dissection).
**Degree of Uterine Convolution** - A measure of anatomical distortion due to adhesions. The degree of uterine convolution was recorded as:
No convolution - Straight lengths of adherent or non-adherent horns which are clearly discerned.
Partly convoluted - Horns have adhesions and 50-75% of the horn length is entangled preventing discernment of straight portions.
Completely convoluted - It is impossible to discern uterine anatomy because the horn is completely entangled.

### Histological and Photographic Procedures

Photographs were taken of the surgical procedure and during the dissection of most of the animals. Uteri and ovaries were retained in 10% neutral buffered formalin.

Animals were excluded from the primary analysis if there were signs of unusual occurrences that may have affected the outcome. Such signs commonly include presence of infection within the abdominal cavity. Any decision to exclude an animal was made prior to inspection of the surgical site and evaluation of adhesions without knowledge of the group assignment or of the presence or extent of adhesions.

### Statistical analysis

The average % extent of adhesions was calculated for the two horns. This average was used to calculate the mean extent of adhesions (+ SEM) for the group, displayed to one decimal place. The comparison of the extent of adhesions in the dyed and non dyed fibrin glue groups was made by constructing a 95% one-sided upper confidence limit for the difference (with dye minus without dye), assuming normality (i.e. based on Student's t-test). As per the protocol, if this confidence limit is below 20 percentage points, fibrin glue plus indigo carmine was to be declared non-inferior to non dyed fibrin glue.

Comparison with the control group was made to demonstrate assay sensitivity, and was made using Student's t-test. The incidence of adhesions was compared using Fisher's Exact Test, and the tenacity and degree of uterine convolution was compared using the chi2 test. For all tests, the level of statistical significance was taken as p<0.05.

**Results**

Both formulations were easy to handle and apply. There was no obvious effect of any of the formulations on the healing of the abdominal wall incision.

All animals recovered uneventfully from the surgical procedure and gained body weight during the study period. The body weight changes are presented in Table 13 below.

**Table 13: Body Weight Changes**

| Group | Mean Wt Change | N | SEM |
|---|---|---|---|
| Fibrin glue | 0.25 | 13 | 0.02 |
| Fibrin glue + dye | 0.23 | 11 | 0.02 |
| Control | 0.25 | 5 | 0.04 |

It is apparent that there were no differences in weight change between the study groups.

### Effect of dyed and undyed fibrin glue formulation on adhesion formation

Results are summarized in Table 14 below.

**Table 14: Effect of Dyed and non dyed fibrin glue formulation on adhesion formation**

| **Group** | **Extent %¹** | **p t-test²** | **Adhesion Free³** | **Grade⁴** | **Conv⁵** | **Mat⁶** | **N⁷** |
|---|---|---|---|---|---|---|---|
| **Fibrin glue** | 9.3 (3.1) | 0.027 | 38%^{§} | 10/15/1^{#} | 25/1/0^{##} | 5/6/2 | 13 |
| **Fibrin glue + dye** | 8.4 (1.4) | 0.027 | 36%^{§§} | 8/12/2^{#} | 22//0/0^{##} | 4/3/4 | 11 |
| **Control** | 42.5 (10.1) | | 10% | 1/2/7 | 8/0/2 | n/a | 5 |

| | | | | | | | |
|---|---|---|---|---|---|---|---|
| 1. % of length of uterine horn with adhesions, mean of left and right horns (SEM) 2. p value for Student's t- test against Control 3. % of uterine horns free of adhesions (Number of uterine horns free of adhsions/total) 4. Number of horns with no adhesions/grade 1 adhesions/ grade 2 adhesions 5. Number of horns with no convolution/partial convolution/full convolution 6. Material remnant small/moderate/large 7. Number of animals ^{#}p ≤ 0.01, χ² test, vs Control; ^{##} p < 0.05, χ² test, vs Control; ^{§} p < 0.088, Fisher's Exact Test, vs Control; ^{§§} p < 0.114, Fisher's Exact Test, vs Control | | | | | | | |

Extensive adhesions were formed in control animals (42.5 ± 10%). Statistically significant reductions in the % extent of adhesion formation were observed for both non dyed and dyed fibrin glue formulations (9.3 ± 3.1%, p = 0.027 and 8.4 ± 1.4%, p = 0.027) as compared to the control group.

Both non dyed and dyed fibrin glue formulations effected statistically significant reductions in the tenacity of adhesions and the degree of uterine convolution compared with controls. Both formulations increased the adhesion free outcome from 10% in controls to 38% (non dyed) and 36% (Dyed), but these differences were not statistically significant.

Comparison of the extent of adhesions in the two fibrin glue groups (dyed and non dyed) showed that the upper confidence limit was 5.44, indicating that both tested group were equally efficient in reducing post-surgical adhesions.

These results show that both fibrin glue formulations (dyed and non dyed) were equally efficient in reducing post surgical adhesions. The addition of dye to the formulation resulted in no noticeable changes in efficacy, handling properties, adverse events or degradation properties.

## Claims

1. A fibrin glue kit for preparing a fibrin glue formulation for application to a surface of a body part of a patient, said kit comprising:
a first separate component required to form the fibrin glue formulation, the first separate component comprising a solution of fibrinogen,
a second separate component required to form the fibrin glue formulation, the second separate component comprising a solution of thrombin which is capable of forming fibrin when it reacts with the fibrinogen, wherein said solution of thrombin further comprises a visualization agent,
**characterized in that** either:
(a) said visualization agent is methylene blue present in said thrombin solution in a concentration range of from 0.01 to 0.05%, and said methylene blue-dyed thrombin solution is protected from light; or
(b) said visualization agent is indigo carmine present in said thrombin solution in a concentration range of from 0.01% to 0.02%.

2. The kit according to claim 1, further comprising a catalyst capable of inducing cross-linking of fibrin.

3. The kit according to claim 1 or 2, wherein the catalyst is a transglutaminase.

4. The kit according to claim 3, wherein the transglutaminase is Factor XIII.

5. The kit according to claim 4, wherein Factor XIII is incorporated in the fibrinogen solution.

## Patentansprüche

1. Fibrinklebstoff-Kit zum Herstellen einer Fibrinklebstoff-Formulierung zum Aufbringen auf eine Oberfläche eines Körperteils eines Patienten, wobei das Kit umfasst:
eine erste gesonderte Komponente, die zum Bilden der Fibrinklebstoff-Formulierung erforderlich ist, wobei die erste gesonderte Komponente eine Lösung von Fibrinogen umfasst,
eine zweite gesonderte Komponente, die zum Bilden der Fibrinklebstoff-Formulierung erforderlich ist, wobei die zweite gesonderte Komponente eine Lösung von Thrombin umfasst, das fähig ist, Fibrin zu bilden, wenn es sich mit dem Fibrinogen umsetzt, wobei die Lösung von Thrombin ferner ein Visualisierungsmittel umfasst,
**dadurch gekennzeichnet, dass** entweder
(a) das Visualisierungsmittel Methylenblau ist, das in der Thrombinlösung in einem Konzentrationsbereich von 0,01 bis 0,05 % vorhanden ist, und die Methylenblau-gefärbte Thrombinlösung vor Licht geschützt ist; oder
(b) das Visualisierungsmittel Indigokarmin ist, das in der Thrombinlösung in einem Konzentrationsbereich von 0,01 % bis 0,02 % vorhanden ist.

2. Kit gemäß Anspruch 1, ferner umfassend einen Katalysator, der fähig ist, Vernetzung von Fibrin zu induzieren.

3. Kit gemäß Anspruch 1 oder 2, wobei der Katalysator eine Transglutaminase ist.

4. Kit gemäß Anspruch 3, wobei die Transglutaminase Faktor XIII ist.

5. Kit gemäß Anspruch 4, wobei Faktor XIII der Fibrinogenlösung einverleibt ist.

## Revendications

1. Kit de colle de fibrine pour l'élaboration d'une formule de colle de fibrine pour application à la surface d'une partie de l'organisme d'un patient, ledit kit comprenant :
un premier composant individuel nécessaire à la formation de la formule de colle de fibrine, le premier composant individuel comprenant une solution de fibrinogène,
un second composant individuel nécessaire à la formation de la formule de col de fibrine, le second composant individuel comprenant une solution de thrombine pouvant former de la fibrine lorsqu'elle réagit avec le fibrinogène, ladite solution de thrombine comprenant en outre un agent de visualisation, caractérisé en ce qui :
(a) soit ledit agent de visualisation est le bleu de méthylène présent dans ladite solution de thrombine dans un intervalle de concentrations comprises entre 0,01 et 0,05 %, et ladite solution de thrombine colorée au bleu de méthylène est protégée de la lumière ;
(b) soit ledit agent de visualisation est le carmin d'indigo présent dans ladite solution de thrombine dans un intervalle de concentrations comprises entre 0,01 % et 0,02 %.

2. Kit conforme à la revendication 1, comprenant en outre un catalyseur pouvant induire la réticulation de la fibrine.

3. Kit conforme à la revendication 1 ou 2, où le catalyseur est une transglutaminase.

4. Kit conforme à la revendication 3, où la transglutaminase est le Facteur XIII.

5. Kit conforme à la revendication 4, où le Facteur XIII est incorporé dans la solution de fibrinogène.
